(19) Europäisches Patentamt
European Patent Office
Office européen des brevets

(11) EP 0 795 334 B1

(12) # EUROPÄISCHE PATENTSCHRIFT

(45) Veröffentlichungstag und Bekanntmachung des Hinweises auf die Patenterteilung:
**01.02.2006 Patentblatt 2006/05**

(51) Int Cl.:
*A61K 47/48* (2006.01) *A61P 35/00* (2006.01)

(21) Anmeldenummer: **97103162.0**

(22) Anmeldetag: **27.02.1997**

(54) **Neuartige Prodrugs für die Therapie von Tumoren und entzündlichen Erkrankungen**

Prodrugs for the treatment tumors and inflammatory diseases

Prodrogues pour le traitement des tumeurs et des maladies inflammatoires

(84) Benannte Vertragsstaaten:
**AT BE CH DE DK ES FI FR GB GR IE IT LI LU NL PT SE**
Benannte Erstreckungsstaaten:
**SI**

(30) Priorität: **12.03.1996 EP 96103866**

(43) Veröffentlichungstag der Anmeldung:
**17.09.1997 Patentblatt 1997/38**

(73) Patentinhaber: **Sanofi-Aventis Deutschland GmbH**
**65929 Frankfurt am Main (DE)**

(72) Erfinder:
- **Bosslet, Klaus, Dr.**
  **35037 Marburg (DE)**
- **Czech, Jörg, Dr.**
  **35041 Marburg (DE)**
- **Gerken, Manfred, Dr.**
  **35041 Marburg (DE)**
- **Straub, Rainer, Dr.**
  **35037 Marburg (DE)**
- **Monneret, Claude, Dr.**
  **75012 Paris (FR)**
- **Florent, Jean-Claude, Dr.**
  **91940 Les Ulis (FR)**
- **Schmidt, Frederic**
  **94300 Vincennes (FR)**

(56) Entgegenhaltungen:
**EP-A- 0 501 215** **EP-A- 0 511 917**
**EP-A- 0 595 133** **EP-A- 0 642 799**
**WO-A-90/07929** **WO-A-92/19639**
**CA-A- 2 081 281**

- **BRITISH JOURNAL OF CANCER, Bd. 72, 1995, Seiten 1083-1088, XP000567843 DC BLAKEY ET AL: "Anti-tumour effects of an antibody-carboxypeptidase G2 conjugate in combination with phenol mustard prodrugs"**
- **CANCER RESEARCH, Bd. 54, Nr. 8, 15.April 1994, USA, Seiten 2151-2159, XP000196209 K. BOSSLET ET AL: "Tumor-selective Prodrug Activation by Fusion Protein-mediated Catalysis"**
- **BRITISH JOURNAL OF CANCER, Bd. 56, Nr. 5, November 1987, GB, Seiten 531-532, XP000196210 K.D. BAGSHAWE: "Antibody directed enzymes revive anti-cancer prodrugs concept"**
- **CANCER RESEARCH, Bd. 55, Nr. 21, 1.November 1995, USA, Seiten 4808-4812, XP000196212 Q. T. TRINH ET AL: "Enzyme/prodrug Gene THerapy: Comparison of Cytosine Deaminase/5-Fluorocytosine versus Thymidine Kinase/Ganciclovir Enzyme/Prodrug Systems in a Human Colorectal Carcinoma Cell Line"**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 795 334 B1

## Beschreibung

Die Wirkung von Pharmaka (Drugs) besteht sehr häufig darin, daß bei bestimmten Erkrankungen pathologisch überexprimierte Enzyme, Cytokine oder andere Faktoren in ihrer krankheitsfördernden Aktivität gehemmt werden. Allerdings erstreckt sich die hemmende Wirkung der Drugs nicht nur auf die pharmakologischen Zielstrukturen (Enzym, Cytokin, Faktor) im erkrankten Gewebe, sondern sie hemmen auch die in gesunden Geweben vorkommenden Aktivitäten. Hieraus ergeben sich die bei vielen Drugs beobachteten unerwünschten Nebenwirkungen. Um die Nebenwirkungen von Drugs zu mildern, wurden experimentelle Systeme entwickelt die eine selektivere Freisetzung von Drugs im erkrankten Gewebe erlauben. Im folgenden werden solche Systeme kurz beschrieben:

Das ADEPT-System (Antibody directed enzyme prodrug therapy, Bagshawe 1987, Br. J. Cancer. 56: 531-532) ist ein Zwei-Schritt-System, bei dem im ersten Schritt ein Antikörperenzymkonjugat(AEK) iv injiziert wird. Das AEK wird aufgrund seiner Tumorselektivität im Tumor zurückgehalten, aber im Laufe von 2-7 Tagen aus den gesunden Geweben ausgeschieden. Die im zweiten Schritt iv injizierte Prodrug (eine ungiftige Drug-Vorstufe) wird im Tumor durch die enzymatische Aktivität des AEK zur giftigen Drug aktiviert. Als Konsequenz dieser tumorspezifischen Prodrug Aktivierung werden erhöhte Drug-Konzentrationen im Tumor(5-50fach) und niedrigere Drug Konzentrationen im gesunden Gewebe im Vergleich zur Standard Therapie beoabachtet. Dies hat eine bessere Verträglichkeit sowie überlegene therapeutische Effekte in humanen Tumorxenograftmodellen zur Folge.(Sharma, S. K. et al. 1991, Disease Markers 9: 225-231)

Ähnlich wie das ADEPT-System arbeitet das FMPA-Konzept (Fusion protein mediated prodrug activation), bei dem an Stelle des xenogenen und demzufolge immunogenen AEK ein nicht immunogenes humanes Fusionsprotein zur tumorselektiven Prodrug-Aktivierung eingesetzt wird.(Bosslet et al 1994, Cancer Res. 54: 2151-2159)

Auch im VDEPT-System (Vector dependent enzyme prodrug therapy, Trinh et al Cancer Res. 55: 4808-4812), einem zwei Schritt gentherapeutischen Ansatz, werden Prodrugs tumorselektiv nach Injektion eines Vektors und Expression eines Strukturgens, welches für ein Enzym codiert, aktiviert.

Eine endogene Aktivierung von Prodrugs (Glucuronyl-Spacer-Anthrazyclin, Jacquessy et al 1991, WO 92/19639) in nekrotischen Tumoren und entzündlichen Prozessen verbunden mit starken antitumoralen und antientzündlichen pharmakologischen Effekten wurde von Bosslet et al. 1994, Cancer Res. 54: 2151-2159 und 1995, Tumor Targeting 1. 45-50 zum ersten Mal als PMT (Prodrug Monotherapie) beschrieben. Bei der pharmakologischen Bearbeitung des PMT-Systems zeigte sich, daß sowohl die Chemie des selbst eliminierenden Spacers als auch die Hydrophilie und molare Zytotoxizität der Drug-Komponente in der Prodrug von entscheidender Bedeutung für die in vivo Effizienz ist. Eine weitere Effizienzsteigerung der PMT wurde in Kombination mit Substanzen beobachtet, die Nekrosen induzieren.(EP 0696456 A2) Vor allem die Verwendung von Antikörperkonjugaten, mit Spezifität für den VEGF/VEGF-Rezeptorkomplex, kovalent verbunden mit koagulatorischen Proteinen, wie zum Beispiel dem verkürzten Tissuefactor, zeigen in pharmakologischen in vivo Modellen kombiniert mit geeigneten Prodrugs besonders gute Wirksamkeit.

In der Europäischen Patentanmeldung mit der Publikationsnummer 0 642 799 A1 werden enzymatisch spaltbare Prodrugs mit reduzierter Michaelis-Menten-Konstante beschrieben. In der Kanadischen Patentanmeldung, Nummer 2,081,281, werden Konjungate beschrieben, enthaltend einen monoklonalen Antikörper, der auf der Oberfläche von Krebszellen binden kann, und ein Enzym β-Glucuronidase. Diese Konjungate können Glucuronid-Prodrugs auf der Oberfläche von Krebszellen aktivieren.

Überraschenderweise ist es uns gelungen, Prodrugs zu synthetisieren, die in vivo, nach entsprechender endogener enzymatischer Aktivierung noch wesentlich wirksamer sind als die in EP 0511917 A1 und EP 0595133 A2 beschriebenen Prodrugs. Diese überlegene Wirksamkeit ist einerseits bedingt durch die neuartige und vorteilhafte Chemie des Spacers, andererseits aber auch durch die hohe molare Zytotoxizität der verwendeten Drug-Komponente. Die neue vorteilhafte Chemie des Spacers zeichnet sich dadurch aus, daß insbesondere bei Wirkstoffen, die über eine Hydroxylgruppe an den Spacer gebunden sind, eine Freisetzung des Wirkstoffes nach enzymatischer Spaltung des Glycosylrestes durch Zyklisierung und Abspaltung des Spacers erfolgt. Hierdurch wird eine verbesserte Stabilität der Produgs bei gleichzeitiger guter enzymatischer Spaltbarkeit erreicht. Die erfindungsgemäßen Prodrugs sind ferner stabiler unter physiologischen Bedingungen als bekannte Produgs, weil sie nicht so schnell den Wirkstoff freisetzen.

Die Erfindung betrifft Prodrugs der Formel I

$$\text{Glykosyl-Y[-C(=Y)-X-]}_p\text{-W(R)}_n\text{-Z-C(=Y)-Wirkstoff} \qquad \text{(I)}$$

und/oder physiologisch verträgliche Salze der Verbindung der Formel I, wobei Glykosyl für ein enzymatisch abspaltbares Poly-, Oligo- oder Monosaccharid steht,

W für

1) 5- bis 14-gliedriger aromatischer Rest,
2) Naphthyl,
3) Indenyl,
4) Anthryl,
5) Phenanthryl,
6) ein 5- bis 14-gliedriger heterozylischer Rest mit 1, 2, 3 oder 4 Heteroatomen aus der Gruppe Sauerstoff, Stickstoff und Schwefel,
7) ($C_1$-$C_6$)-Alkyl,
8) ($C_2$-$C_6$)-Alkenyl,
9) ($C_3$-$C_6$)-Cycloalkyl oder
10) Phenyl steht,

R für

1) Wasserstoffatom,
2) ($C_1$-$C_4$)-Alkyl,
3) Phenyl,
4) Methoxy,
5) Carboxy,
6) Methyloxycarbonyl,
7) -CN,
8) -OH,
9) -$NO_2$,
10) Halogen wie Fluor, Chlor oder Brom,
11) Sulfonyl,
12) Sulfonamid oder
13) Sulfon-($C_1$-$C_4$)-alkylamid steht,

p für Null oder 1 steht,
n für Null, 1, 2 oder 3 steht,
X für

1) Sauerstoffatom,
2) -NH-,
3) Methylenoxy,
4) Methylenamino,
5) Methylen-($C_1$-$C_4$)-alkylamino,
6) ($C_1$-$C_4$)-Alkylamino oder
7) ($C_3$-$C_6$)-Cycolalkylamino steht,

Y für Sauerstoffatom oder -NH- steht,
Z für

1) ($C_1$-$C_4$)-Alkylamino,
2) -$N(CH_3)$-,
3) -$C(CH_3)_2$-NH-,
4) -$CH(CH_3)$-NH-,
5) -$C(CH_3)_2$-$N(R^2)$-, worin $R^2$ ($C_1$-$C_4$)-Alkyl bedeutet, oder
6) -NH-, wenn W für ($C_1$-$C_6$)-Alkyl, steht, und

Wirkstoff für eine Verbindung mit biologischer Wirkung steht, die über einen Sauerstoffrest, primären oder sekundärer Aminorest oder einen Iminorest gebunden ist.

Wenn n für die ganze Zahl 2 oder 3 steht dann stehen die Reste R unabhängig voneinander für die unter R1) bis R13) genannten Bedeutungen.
Unter dem Begriff Alkyl oder Alkenyl werden Reste verstanden deren Kohlenstoffkette geradkettig, verzweigt oder

cyclisch sein kann; die Doppelbindungen können mehrfach vorkommen. Cyclische Alkylreste sind beispielsweise 3- bis 6-gliedrige Monocyclen wie Cyclopropyl, Cyclobutyl, Cyclopentyl oder Cyclohexyl. Zu dem Begriff "5- bis 14-gliedriger heterozyklischer Rest mit 1, 2, 3 oder 4 Heteroatomen aus der Gruppe Sauerstoff, Stickstoff und Schwefel" gehören z.B. Reste, die sich von Pyrrol, Azepin, Pyrrolin, Pyridin, Imidazol, Pyrimidin, Triazin, Furan, 1,2-Diazepin, Oxazol, Pyrazin, lsoxazol, Isoxazolin,Thiazol, Isothiazol, Isothiazolidin, Indol, Chinolin, Isochinolin, Benzimidazol, Indazol, Purin, Pteridin, Thiopyran oder Thiophen ableiten.

Geeignete physiologisch verträgliche Salze der Verbindung der Formel I sind beispielsweise Alkali-, Erdalkali- und Ammoniumsalze einschließlich solcher von organischen Ammoniumbasen.

Unter dem Begriff Monosaccharid werden Reste wie D-Glucuronyl, L-Iduronyl, D-Glucopyranosyl-, D-Galactopyranosyl, N-Acetyl-D-glucosaminyl-, N-Acetyl-D-galactosaminyl-, D-Mannopyranosyl- oder L-Fucopyranosyl verstanden. Oligo- oder Polysaccharide bestehen aus 2 bis 20 der obengenannten Monosaccharide, die alpha- oder beta-O-glykosidisch miteinander verbunden sind. Die Bindung zwischen dem Monosaccharid und den Rest Y ist alpha- oder beta-glykosidisch. Geeignete Enzyme die die Spaltung des Glycosylrestes vom Rest Y bewirken sind Induronidase, Glucosidase, Galactosidase, N-Acetyl-D-glucosaminidase, N-Acetyl-D-galactosaminidase, Mannosidase, Fucosidase oder Glucuronidase, bevorzugt β-Glucuronidase.

Als Wirkstoff sind Verbindungen geeignet wie Anthrazyklin, vorzugsweise Doxorubicin, 4'-epi-Doxorubicin, 4- oder 4'-Desoxy-doxorubicin oder eine Verbindung vorzugsweise ausgewählt aus der Gruppe Etoposide, N-Bis(2-chlorethyl)-4-hydroxyanilin, 4-Hydroxycyclophosphamid, Vindesin, Vinblastin, Vincristin, Terfenadin, Terbutalin, Fenoterol, Salbutamol, Muscarin, Oxyphenbutazon, Salicylsäure, p-Aminosalicylsäure, 5-Fluorouracil, 5-Fluorocytidin, 5-Fluorouridin, Methotrexat, Diclofenac, Flufenaminsäure, 4-Methylaminophenazon, Theophyllin, Nifedipin, Mitomycin C, Mitoxantron, Camptothecin, m-AMSA, Taxol, Nocodazol, Colchicin, Cyclophosphamid, Rachelmycin, Cisplatin, Melphalan, Belomycin, Stickstoff-Senfgas, Phosphoramid-Senfgas, Quercetin, Genistein, Erbstatin, Tyrphostin, Rohitukin-Derivat ((-)-cis-5,7-Dihydroxy-2-(2-chlorphenyl)-8-[4-(3-hydroxy-1-methyl)-piperidinyl]-4H-1-benzopyran-4-on; EP 0 366 061), Retinolsäure, Buttersäure, Phorbolester, Aclacinomycin, Progesteron, Buserelin, Tamoxifen, Mifepriston, Onapriston, N-(4-aminobutyl)-5-chloro-2-naphthalen-sulfonamid, Pyridinyloxazol-2-on, Quinolyloxazolone-2-on, lsoquinolyloxazolone-2-on, Staurosporin, Verapamil, Forskolin, 1,9-Dideoxyforskolin, Quinin, Quinidin, Reserpin, 18-O-(3,5-dimethoxy-4-hydroxybenzoyl)-reserpat, Lonidamin, Buthioninsulfoximin, Diethyldithiocarbamat, Cyclosporin A, Azathioprin, Chlorambucil, N-(4-Trifluormethyl)-phenyl-2-cyano-3-hydroxycrotonsäureamid (WO 91 17748), 15-Deoxyspergualin, FK 506, Ibuprofen, Indomethacin, Aspirin, Sulfasalazin, Penicillinamin, Chloroquin, Dexamethason, Prednisolon, Lidocain, Propafenon, Procain, Mefenaminsäure, Paracetamol, 4-Aminophenazon, Muskosin, Orciprenalin, Isoprenalin, Amilorid, p-Nitrophenylguanidinbenzoat oder deren durch eine oder mehrere Hydroxy-, Amino- oder Iminogruppen zusätzlich substituierte Derivate.

Bevorzugt werden Prodrugs, worin der Wirkstoff ein Zytostatikum ist, ein Antimetabolit ist, daß der Wirkstoff 5-Fluorouracil, 5-Fluorocytidin, 5-Fluorouridin, Cytosinarabinosid oder Methotrexat ist, daß der Wirkstoff eine in die DNA interkalierende Substanz ist, daß der Wirkstoff Doxorubicin, Daunomycin, Idarubicin, Epirubicin oder Mitoxantron ist, daß der Wirkstoff die Topoisomerase I und II hemmt, daß der Wirkstoff Camptothecin, Etoposid oder m-AMSA ist, daß der Wirkstoff ein Tubulinhemmer ist, daß der Wirkstoff Vincristin, Vinblastin, Vindesin, Taxol, Nocodazol oder Colchicin ist, daß der Wikrstoff ein Alkylanz ist, daß der Wirkstoff Cyclophosphamid, Mitomycin C, Rachelmycin, Cisplatin, Phosphoramid-Senfgas, Melphalan, Bleomycin, Stickstoff-Senfgas oder N-Bis-(2-chlorethyl)-4-hydroxyanilin ist, daß der Wirkstoff Neocarzinostatin, Calicheamicin, Dynemicin oder Esperarmicin A ist, daß der Wirkstoff eine die Ribosomen inaktivierende Verbindung ist, daß der Wirkstoff Verrucarin A ist, daß der Wirkstoff ein Tyrosinphosphokinaseinhibitor ist, daß der Wirkstoff Quercetin, Genistein, Erbstatin, Tyrphostin oder ein Rohitukin-Derivat ist, daß der Wirkstoff ein Differenzierungsinduktor ist, daß der Wirkstoff Retinolsäure, Buttersäure, Phorbolester oder Aclacinomycin ist, daß der Wirkstoff ein Hormon, Hormonagonist oder Hormonantagonist ist, daß der Wirkstoff Progesteron, Buserelin, Tamoxifen, Mifepriston oder Onapriston ist, daß der Wirkstoff eine Substanz ist, welche die pleiotrope Resistenz gegenüber Zytostatika verändert, daß der Wirkstoff ein Calmodulin-Inhibitor ist, daß der Wirkstoff ein Proteinkinase C-Inhibitor ist, daß der Wirkstoff ein P-Glykoprotein-Inhibitor ist, daß der Wirkstoff ein Modulator der mitochondrial gebundenen Hexokinase ist, daß der Wirkstoff ein Inhibitor der γ-Glutamylcysteinsynthetase oder der Glutathion-S-transferase ist, daß der Wirkstoff ein Inhibitor der Superoxiddismutase ist, daß der Wirkstoff einen Inhibitor des durch den MAk Ki67 definierten proliferationsassoziierten Proteins im Zellkern einer sich teilenden Zelle darstellt, daß der Wirkstoff eine Substanz ist, die immunosuppressive Effekte ausübt, daß der Wirkstoff ein Standardimmunsuppressivum ist, daß der Wirkstoff ein Makrolid ist, daß der Wirkstoff Cyclosporin A, Rapamycin, FK 506 ist, daß der Wirkstoff Azathioprin, Methotrexat, Cyclophosphamid oder Chlorambucil ist, daß der Wirkstoff eine Substanz ist, die antiinflammatorische Wirkung hat, daß der Wirkstoff eine nicht stereoidale antiinflammatorische Substanz ist, daß der Wirkstoff eine "slow acting antirheumatic drug" ist, daß der Wirkstoff ein Steroid darstellt, daß der Wirkstoff eine Substanz ist, die antiphlogistische, analgetische oder antipyretische Wirkung hat, daß der Wirkstoff ein Derivat einer organischen Säure darstellt, daß der Wirkstoff ein nicht saures Analgetikum/Antiphlogistikum darstellt, daß der Wirkstoff Oxyphenbutazon ist, daß der Wirkstoff ein Lokalanästhetikum ist, daß der Wirkstoff ein Antiarrhythmikum ist, daß der Wirkstoff ein $Ca^{++}$ Antagonist ist, daß der Wirkstoff

ein Antihistaminikum ist, daß der Wirkstoff ein Sympathomimetikum ist oder daß der Wirkstoff eine Substanz mit inhibitorischer Wirkung auf die humane Urokinase ist; und Derivate der obengenannten Wirkstoffe, wobei der Wirkstoff über einen Sauerstoffrest, -NH-Rest oder Iminorest an den Rest Y der Verbindung der Formel I gebunden ist.
Der Wirkstoff ist auch vorzugsweise die in den Beispielen genannte Stickstofflostverbindung, Quinin oder Dipyridamol.

Bevorzugt sind Prodrugs, wobei
Glykosyl für eine enzymatisch abspaltbare Glukuronsäure steht,

- W für Phenyl steht,
- R für Wasserstoffatom, CN, Nitro, Fluor, Chlor, Brom steht,
- p für Null ist,
- n für eine ganze Zahl Null, 1 oder 2 steht,
- Y für Sauerstoffatom steht,
- Z für $-N(CH_3)-$, $-C(CH_3)_2-NH-$, $-CH(CH_3)-NH-$, $-C(CH_3)_2-N(C_1-C_4)alkyl)-$, $-CH(CH_3)-N(C_1-C_4)alkyl)-$ steht und

Wirkstoff eine über eine Hydroxy-, Amino- oder Iminogruppe verknüpfte Verbindung mit biologischer Wirkung ist.

Insbesondere bevorzugt sind die Verbindungen
2-[N-Methyl-N-[(4-(N,N'-bis-(2-chloroethyl)amino)phenyloxycarbonyl]amino]-4-nitrophenyl-β-D-glucuronsäure,
2-[N-Methyl-N-[(4-(N,N'-bis-(2-iodoethyl)amino)phenyloxycarbonyl]amino]-4-nitrophenyl-β-D-glucuronsäure,

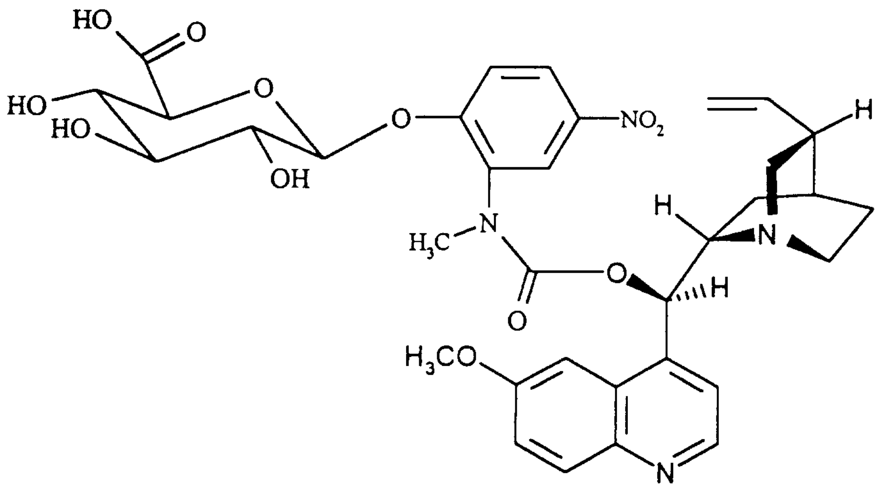

oder

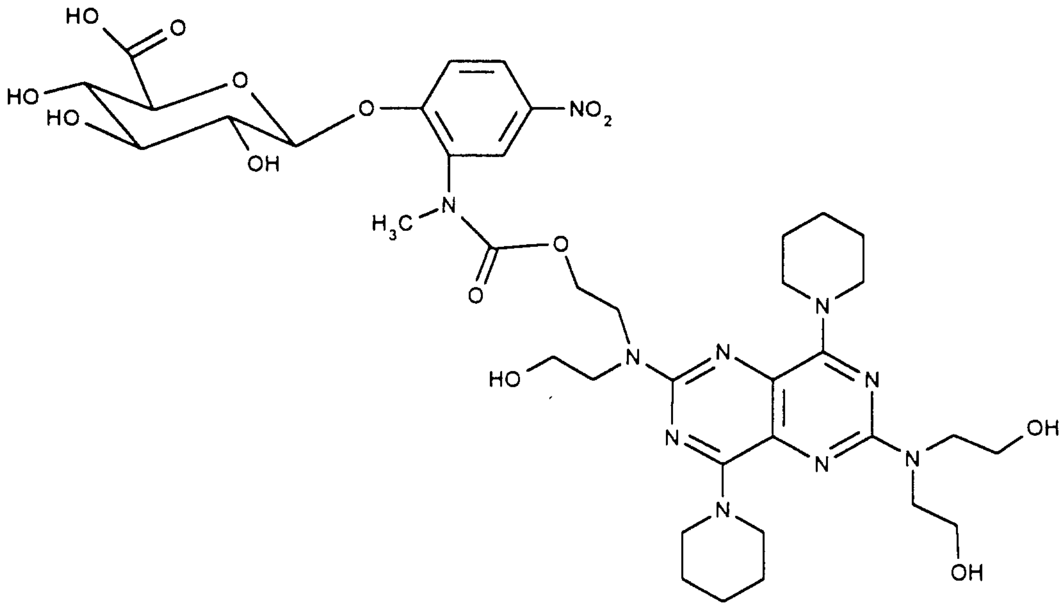

Die Erfindung betrifft auch ein Verfahren zur Herstellung des Prodrugs der Formel I, das dadurch gekennzeichnet ist, daß man eine Verbindung der Formel II,

$$\text{Glykosyl-Y[-C(=Y)-X-]}_p\text{-W(R)}_n\text{-Z} \qquad \text{(II)}$$

worin die Reste Glykosyl, Y, X, p, W, R, n und Z die in Formel I genannte Bedeutung haben, mit Wirkstoff, der einen aktivierten Carboxyl-, Amino- oder Iminorest hat, umsetzt, wobei die Umsetzung in Gegenwart eines Lösungsmittels aus der Gruppe Acetonitril, Dioxan, Tetrahydrofuran, Dichlormethan, Dimethylformamid, Aceton erfolgt und anschließend die Schutzgruppen hydrolytisch abgespalten werden.

Die Aktivierung des Wirkstoffs erfolgt beispielsweise nach H.J. Marley, Chem. Soc. Chem. Communication (1987) Seiten 112-113 oder nach H. Hagemann Angew. Chem., 93 (1981) Seiten 813-814. Die Abspaltung der Schutzgruppen erfolgt beispielsweise mit Alkalilauge, Alkalicarbonat, Alkalicyanid, Bariumoxid, Piperidin oder Morpholin in Gegenwart von Methanol, Ethanol oder Wasser.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I, wobei die Reste Glykosyl, Y, X, p, W, R, n, Z und Wirkstoff wie oben definiert sind, zusammen mit einem pharmazeutisch geeignetem und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen hervorragend zur Prophylaxe und Therapie all solcher Erkrankungen oder Störungen, an deren Verlauf intrazelluläre Enzyme, die den Glycosylrest spalten können überexpremiert und/oder freigesetzt werden oder durch Zellzerstörung zugänglich werden. Dies sind vor allem Erkrankungen wie Krebs, Autoimmunerkrankungen oder entzündliche, immunologisch oder stoffwechselbedingte akute und chronische Arthritiden und Arthropathien, insbesondere Tumorerkrankungen und rheumatische Arthritis.

Die Erfindung betrifft ferner die Verwendung der Verbindung der Formel I zur Herstellung von Arzneimitteln zur Prophylaxe und Therapie von Krebserkrankungen, Autoimmunerkrankungen und chronisch entzündlicher Erkrankungen wie rheumatische Arthritis.

Die Erfindung betrifft auch ein Verfahren zur Herstellung eines Arzneimittels, das dadurch gekennzeichnet ist, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete Zubereitungsformen sind beispielsweise injizierbare Lösungen, bei deren Herstellung übliche Hilfsmittel, wie Trägerstoffe, Binde-, Quellungs- oder Schmiermittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polethylenglykol und Lösungsmittel wie etwa steriles Wasser und ein- oder mehrwertige Alkohole wie Glycerin, genannt. Es können auch Liposomen oder humane Proteine als Träger verwendet werden.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei Injektionslösungen kann diese Dosis für einen erwachsenen etwa 70 kg schweren Patienten bis zu etwa 10g, bevorzugt jedoch etwa von 3 g bis 5 g betragen. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Die erfindungsgemäßen Prodrugs lassen sich auch bei allen nicht onkologischen Erkrankungen einsetzen, bei denen Makrophagen, Granulozyten und Thrombozyten, insbesondere in aktiviertem Zustand vorkommen. In aktiviertem Zustand scheiden die oben erwähnten Zellen vornehmlich intrazelluläre Enzyme aus, die eine ortsspezifische Aktivierung der erfindungsgemäßen Prodrugs ermöglicht.

In der onkologischen Indikation erfolgt die Aktivierung der erfindungsgemäßen Prodrugs durch aus sterbenden Tumorzellen freigesetzte intrazelluläre Enzyme. Dieses Phänomen tritt vor allem bei größeren Tumoren auf (Durchmesser mehr als 0,3 cm), aber auch nach Schädigung des Tumors durch Behandlung mit Immuntoxinen, Zytostatika, Bestrahlung, Fusionsproteinen oder Antikörperenzymkonjugaten.
Da der Glykosylanteil der erfindungsgemäßen Prodrugs so gewählt ist, daß er nur von unter pathophysiologischen Bedingungen lokal freigesetzten Enzymen abspaltbar ist, kann die lipophile Drug ebenfalls nur am Zielgewebe freigesetzt werden und dort ihre zytotoxische Wirkung entfalten.
Steigern läßt sich die überlegene Wirkung einer erfindungsgemäßen Prodrug mit zytotoxischer Drugkomponente dadurch, daß sie mit erfindungsgemäßen Prodrugs mit einer anderen zytotoxischen Drugkomponente kombiniert wird. Hierbei sind Prodrugkombinationen von Vorteil, bei denen zytotoxische Komponenten mit unterschiedlichem Wirksamkeitsmechanismus verwendet werden, entsprechend der Poly-Chemotherapie. Besonders geeignet erscheint der Einsatz von Wirkstoffen, die sehr effizient Einzelstrang- und Doppelstrangbrüche in der DNA hervorrufen wie Calicheamicin.
Von besonderem Vorteil sind allerdings erfindungsgemäße Prodrugkombinationen, bei denen das eine Drug zytotoxisches Potential hat und das andere aber die Multi-Drug-Resistenz blockiert.

Ferner betrifft die Erfindung pharmazeutische Zusammensetzungen, enthaltend eine Verbindung der Formel I und Antikörper-Enzym-Konjugate.
Unter Antikörper-Enzym-Konjugaten werden Verbindungen verstanden die über den Antikörperteil spezifisch an Tumorgewebe oder entzündetes Gewebe binden und einen Enzymteil haben der den Glykosylrest der Verbindung der Formel I spalten kann. Beispiele für solche Verbindungen werden in EP 0 501 215, EP 0 390 530 oder EP 0 623 352 beschrieben.

Beispiel 1:Stickstofflostderivat-Prodrug (F373; Verbindung 11)

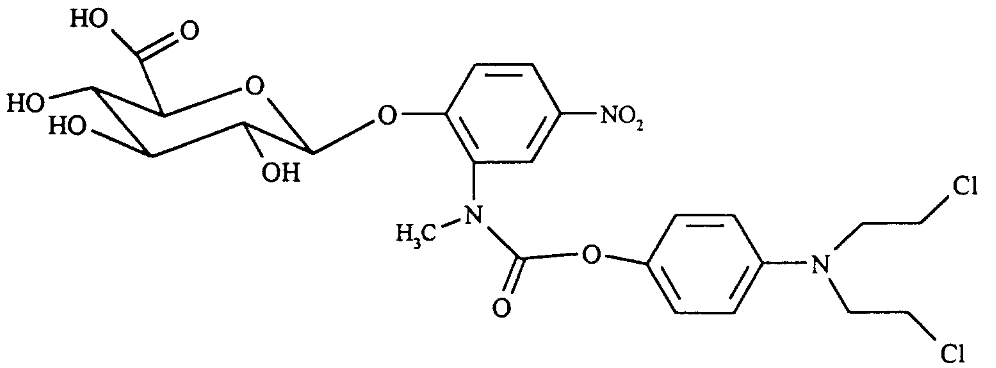

wurde folgendermaßen synthetisiert:

Das Ausgangsmaterial für die Synthese war 2-Amino-4-nitrophenol (Verbindung 1). Verbindung 1 wurde zuerst monomethyliert mit Hilfe von Methyljodid (Verbindung 2) und die Aminofunktion wurde als BOC-Derivat geschützt (Verbindung 3). Die geschützte Glucuronsäure wurde mittels Silberoxidkopplung von Verbindung 3 und dem Bromid (Verbindung 4) unter Erhalt von Verbindung 5 eingeführt. Nach Abspaltung der BOC Schutzgruppe mit HCl wurde das Amin (Verbindung 6) erhalten. Verbindung 7 wurde zum Chloroformat (Verbindung 8) umgesetzt und mit Verbindung 6 zur Verbindung 9 kondensiert. Nach Spaltung der Ester des Glucuronsäureteils der Verbindung 3 in zwei Schritten (MeONa/MeOH,dann wässriges NaOH) wurde über Verbindung 10 die Prodrug (Verbindung 11) erhalten.

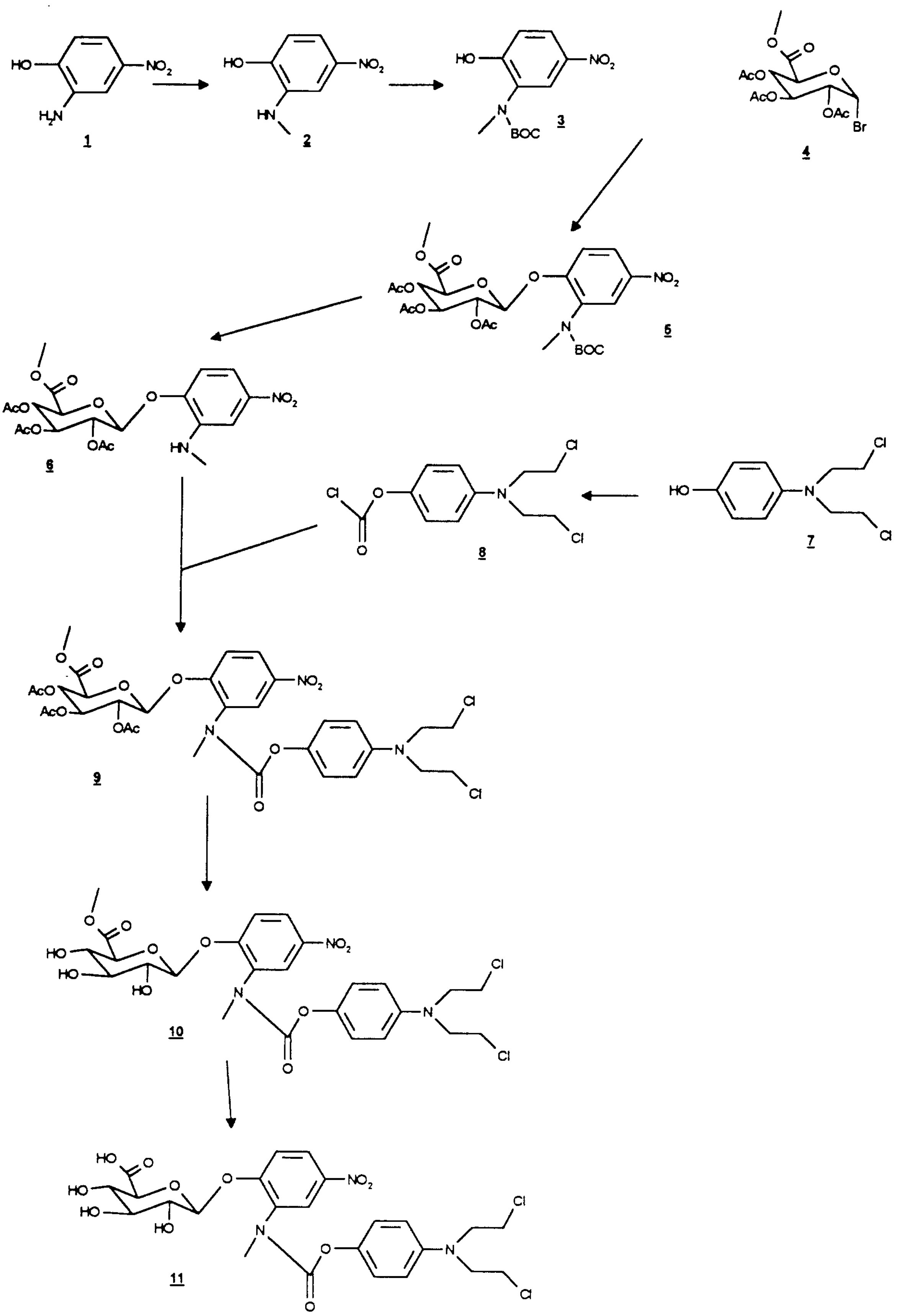

1 2 3 4 5 6 7 8 9 10 11

Verbindung 2:

2-(N-Methylamino)-4-nitrophenol (2)

Zu einer Lösung von 2-Amino-4-nitrophenol (1) (1.54g, 10 mmol) und Methyliodid (1 ml, 16 mmol) in Methanol (10 ml) wurde Triethylamin (2 ml, 14.4 mmol) zugegeben. Nach 1 Stunde bei 40° C wurden weiteres Methyliodid (1 ml) und Triethylamin (1 ml) zugegeben und weitere 2 Stunden bei 40° C gerührt. Die Reaktionsmischung wurde im Vakuum zur Trockene eingeengt, in wässrige 2N Natriumacetatlösung gegeben und mit Essigester extrahiert. Die organische Phase wurde mit Natriumsulfat getrocknet und auf Kieselgel (Laufmittel Dichlormethan /Methanol 95/5) chromatographiert. Ausbeute 880 mg (52%).

| | $C_7H_8N_2O_3$: | | |
|---|---|---|---|
| Berechnet | C:50.02 | H:4.76 | N:16.73 |
| Gefunden | C:50.00 | H:4.80 | N:16.66 |

Schmelzpunkt: 148°C (Toluol)
$^1$H NMR (250 MHz, DMSO):

δ 7.44)(dd,$J_{ortho}$ = 9Hz, $J_{meta}$ = 3.0 Hz, 1H),
7.13 (d,J = 3Hz, 1H),
6.77 (d,J = 9Hz, 1H),
2.76 (s, 3H).

IR (KBr): v($cm^{-1}$) 3363(OH), 1538, 1338($NO_2$).
MS(DCI, $NH_3$):m/z $[M+H]^+$:169.

Verbindung 3:

2-(N-BOC,N-methylamino)-4-nitrophenol (3)

Zu einer Lösung von 2-N-Methylamino-4-nitrophenol (2) (4.18 g, 24.9 mmol) in Tetrahydrofuran (50 ml) wurden Di-tert.-Butyldicarbonat (14 g, 64.15 mmol) Kaliumcarbonat (17 g, 123 mmol) und Wasser (50 ml) gegeben und über Nacht bei Raumtemperatur gerührt. Die Reaktionsmischung wurde mit einer gesättigten wässrigen Ammoniumchloridlösung angesäuert, mit Essigester extrahiert, mit Natriumsulfat getrocknet und im Vakuum eingeengt. Das Rohprodukt wurde zwei Stunden in Methanol (100 ml) mit Kaliumcarbonat (17 g, 123 mmol) gerührt, mit einer gesättigten wässrigen Ammoniumchloridlösung angesäuert, mit Essigester extrahiert, mit Natriumsulfat getrocknet und im Vakuum eingeengt. Das Produkt wurde auf Kieselgel (Laufmittel Dichlormethan / Methanol 97.5/2.5) chromatographiert. Ausbeute 6.2 g (93 %).

| | $C_{12}H_{16}N_2O_5$: | | |
|---|---|---|---|
| Berechnet | C:53.72 | H:6.01 | N:10.44 |
| Gefunden | C:53.64 | H:6.20 | N:10.36 |

Schmelzpunkt: 197°C (Toluol/Petrolether)
$^1$H NMR (250 MHz,DMSO):

δ 8.10-8.00 (2H),
7.03 (d, J = 8.5Hz, 1H),
3.04 (s, 3H),
1.40-1.30 (9H).

IR (KBr): v($cm^{-1}$) 3129 (OH), 1672 (CO), 1529, 1339 ($NO_2$).
MS(DCI, $NH_3$):

m/z [M+H]$^+$:269,
[M+H-$C_4H_8$]$^+$:213,
[M+H-$C_4H_8$OCO]$^+$:169.

Verbindung 5:

2-(N-BOC, N-methylamino)-4-nitrophenyl-2,3,4-tri-O-acetyl-β-D-glucuronsäure methylester (5)

Zu einer Lösung von 2,3,4-Tri-O-acetyl-α-D-glucuronsäure methylester bromid (4) (126 mg, 0.317 mmol) in Acetonitril (5 ml) wurde Silberoxid (0.23 g, 0.992 mmol) und 2-(N-BOC,N-methylamino)-4-nitrophenol (3) zugegeben. Die Reaktionsmischung wurde 1 Stunde bei Raumtemperatur gerührt, über Celite filtriert und im Vakuum eingeengt. Das Produkt wurde auf Kieselgel (Laufmittel Dichlormethan / Methanol 97.5/2.5) chromatographiert. Ausbeute 165 mg (89 %).

| | $C_{25}H_{32}N_2O_{14}$: | | |
|---|---|---|---|
| Berechnet | C:51.37 | H:5.52 | N:4.79 |
| Gefunden | C:51.79 | H:5.72 | N:4.66 |

Schmelzpunkt: 80°C (Toluol/Petrolether)

$$[\alpha]_D^{20} = -39° \text{ (c=1.02 in } CHCl_3).$$

$^1H$ NMR (250 MHz,$CDCl_3$):

δ 8.14 (dd, $J_{ortho}$ = 9Hz, $J_{meta}$ = 2.5 Hz, 1H),
8.15-8.05 (1H),
7.30-7.20 (1H),
5.45-5.30 (4H),
4.25 (d, J = 9 Hz, 1H),
3.73 (s, 3H),
3.13 (s, 3H),
2.15-2.05 (9H),
1.65-1.40 (9H).

IR ($CDCl_3$): ν($cm^{-1}$) 1760 (CO, ester), 1699 (CO, carbamat), 1529, 1349($NO_2$).
MS(DCI, $NH_3$):m/z [M+$NH_4$]$^+$:602.

Verbindung 6:

2-(N-Methylamino)-4-nitrophenyl-2,3,4-tri-O-acetyl-β-D-glucuronsäure methylester (6)

Eine Lösung von 2-(N-BOC,N-methylamino)-4-nitrophenyl-2,3,4-tri-O-acetyl-β-D-glucuronsäure methylester (5) (3 g, 5,13 mmol) in 2,12 M Salzsäure in Essigester (60 ml) wurde 1 Stunde bei Raumtemperatur gerührt. Die Lösung wurde in überschüssige, wässrige, gesättigte Natriumbicarbonatlösung gegeben und mit Essigester extrahiert. Die organische Phase wurde mit Natriumsulfat getrocknet und eingeengt. Das Produkt wurde auf Kieselgel (Laufmittel Dichlormethan /Methanol 97,5/2,5) chromatographiert. Ausbeute 2,14g (86 %), gelber Feststoff.

| | $C_{20}H_{24}N_2O_{12}$: | | |
|---|---|---|---|
| Berechnet | C:49,59 | H:4,99 | N:5,78 |
| Gefunden | C:49,81 | H:5,12 | N:4,80 |

Schmelzpunkt: 120°C (Toluol)

$$[\alpha]_D^{20} = -58° \ (c=1{,}04 \text{ in } CHCl_3).$$

$^1H$ NMR (300 MHz, $CDCl_3$):

δ 7.53 (dd, $J_{ortho}$ = 8.5Hz, $J_{meta}$ = 2.5 Hz, 1H),
7.37 (d, J = 2.5 Hz, 1H),
6.91 (d, J = 8.5 Hz, 1H),
5.45-5.30 (1H),
5.16 (d, J = 7 Hz, b, 1H),
4.50 (d, J = 5 Hz, 1H),
4.24 (d, J = 9 Hz, 1H),
3.75 (s, 3H),
2.90 (d, J = 5 Hz, 1H),
2.10-2.05 (9H).

IR ($CDCl_3$): ν($cm^{-1}$) 3443 (NH), 1758 (CO), 1553, 1346($NO_2$).
MS(DCI, $NH_3$):m/z $[M+H]^+$:485.

Verbindung 8

4-[N,N-bis-(2-chloroethyl)amino]phenyl chloroformat (8)

Phosgen in Toluol (1.93 M) (8 ml, 15.4 mmol) wurde einer Suspension von 4-[N,N-bis(2-chloroethyl)amino]phenol Hydrochlorid (7) (1.85 mmol) in Tetrahydrofuran (30 ml) zugegeben und 30 Minuten bei 0° C gerührt. Nach Zugabe von Triethylamin (1 ml, 7.17 mmol) wurde eine weitere Stunde bei 0° C gerührt. Anschließend wurde die Suspension filtriert und im Vakuum bei Raumtemperatur eingeengt. Flash-Chromatographie an Kieselgel mit Dichlormethan als Laufmittel ergab das Produkt als farblose Flüssigkeit, die sofort in die nächste Reaktion eingesetzt wurde. Ausbeute 70 %.
$^1H$ NMR (250 MHz, $CDCl_3$):

δ 7.10 (d, J = 9 Hz, 2H),
6.66 (d, J = 9 Hz, 2H),
3.73 (t, J = 6.5 Hz, 4H),
3.63 (t, J = 6.5 Hz, 4H).

IR ($CDCl_3$): ν($cm^{-1}$) 1779 (CO), 1514 (aromatisch).
MS(DCI, $NH_3$):

m/z $[M+H]^+$:296,
$[M+2+H]^+$:298.

Verbindung 9:

2-[N-Methyl-N-[4-(N,N'-bis-(2-chloroethyl)amino]phenyloxycarbonyl]amino]-4-nitrophenyl-2,3,4-tri-O-acetyl-β-D-glucuronsäure methylester (9)

Zu einer Lösung von 4-[N,N-bis-(2-chloroethyl)amino)phenyl chloroformat (8) (0.31 g, 1.04 mmol) in Tetrahydrofuran (15 ml), Diisopropylethylamin (0.25 ml, 1.44 mmol) wurde 2-(N-Methylamino)-4-nitrophenyl-2,3,4-tri-O-acetyl-β-D-glucuron-säure methylester (6) (0.50 g, 1.03 mmol) gegeben und 2 Stunden am Rückfluß gekocht. Nach Abkühlen auf Raumtemperatur wurde eingeengt. Das Produkt wurde auf Kieselgel (Laufmittel Dichlormethan / Methanol 97.5/2.5) chromatographiert. Ausbeute 487 mg (64 %).

$C_{31}H_{35}N_3O_{14}Cl_2$:

| | | | | |
|---|---|---|---|---|
| Berechnet | C:50.00 | H:4.74 | N:5.64 | Cl:9.52 |
| Gefunden | C:49.90 | H:4.82 | N:5.62 | Cl:9.78 |

Schmelzpunkt: 101°C (Methanol)

$$[\alpha]_D^{20} = -47° \text{ (c=1.10 in } CHCl_3).$$

$^1H$ NMR (300 MHz, $CDCl_3$):

δ 8.25-8.15 (2H),
7.45-7.35 (1H),
7.25-7.05 (1H),
6.95-6.85 (1H),
6.70-6.55 (2H),
5.45-5.25 (4H),
4.28 (d, J = 9 Hz, 1H),
3.80-3.55 (11H),
3.38(s)2 diastereomere Carbamate(40/60) 3H,
3.27 (s),
2.20-2.00 (9H).

IR ($CDCl_3$): ν($cm^{-1}$) 1760 (CO, ester), 1722 (CO, carbamat),1530, 1350($NO_2$).
MS(DCI, $NH_3$):

m/z $[M+H]^+$:744; $[M+2+H]^+$:746,
$[M+Na]^+$:766, $[M+2+Na]^+$:768.

Verbindung 10:

2-[N-Methyl-N-[(4-(N,N'-bis-(2-chloroethyl)amino)phenyloxycarbonyl]amino]-4-nitrophenyl-β-D-glucuronsäure methylester (10)

Zu einer Suspension von 2-[N-Methyl-N-[4-(N,N'-bis-(2-chloroethyl)amino)phenyloxycarbonyl]amino]-4-nitrophenyl-2,3,4-tri-O-acetyl-β-D-glucuronsäure methylester (9) (68 mg, 0.0915 mmol) in Methanol (5 ml) wurde bei -15°C Natriummethylat (2 mg, 0.037 mmol) gegeben und 6 Stunden bei -15°C gerührt. Nach Neutralisation mit Ionenaustauscher (Amberlite IRC-50 S) und Filtration wurde die Lösung eingeengt und auf Kieselgel mit Essigester als Laufmittel chromatographiert. Ausbeute 50 mg (89%).
$C_{25}H_{29}N_3O_{11}Cl_2$
$^1H$ NMR (300 MHz, $CDCl_3$):

δ 8.22 (sl, 1H),
8.16 (d, J = 8.5 Hz, 1H),
7.25 (d, 1H),
7.15-6.90 (2H),
6.80-6.45 (2H),
5.06 (1H),
4.09 (1H),
4.20-3.15 (17H).

IR ($CDCl_3$): ν($cm^{-1}$) 3601, 3448 (OH), 1714 (CO), 1528, 1349 ($NO_2$).
MS(ES):m/z $[M+Na]^+$:640,$[M+2+Na]^+$:642.

Verbindung 11:

2-[N-Methyl-N-[(4-(N,N'-bis-(2-chloroethyl)amino)phenyloxycarbonyl]amino]-4-nitrophenyl-β-D-glucuronsäure (11)

Zu einer Lösung von Verbindung 10 (50 mg, 0.0809 mmol) in Aceton (4 ml) wurden bei -15°C 0.3 ml einer 1 N wässrigen Natriumhydroxidlösung gegeben. Die Mischung wurde 2 Stunden bei - 15°C gerührt, mit 1 N wässrigen Salzsäure neutralisiert und im Vakuum eingeengt (T < 40°C). Das Produkt wurde auf Kieselgel mit Acetonitril/Wasser

(9/1) chromatographiert. Die Ausbeute betrug 45 mg (89 %).
$C_{24}H_{27}N_3O_{11}Cl_2$
$^1H$ NMR (250 MHz,$CD_3OD$):

δ 8.30 (sl, 1H),
8.24 (dd,$J_{ortho}$ = 9Hz, $J_{meta}$ = 2.5 Hz, 1H),
7.52 (d, J = 9Hz, 1H),
6.99 (d, J = 9Hz, 2H),
6.69 (d, J = 9Hz, 2H),
5.23 (1H),
3.89 (d,J = 9Hz, 1H),
3.80-3.33 (1H).

IR (KR): ν($cm^{-1}$) 3418(OH), 1705(CO), 1516, 1349($NO_2$).
MS(ES):

m/z [M-H]$^+$:603
[M+2-H]$^+$:605.

Beispiel 2

2-[N-Methyl-N-[(4-(N,N'-bis-(2-iodoethyl)amino)phenyloxycarbonyl]amino]-4-nitrophenyl-β-D-glucuronsäure (12)

(Verbindung 12):

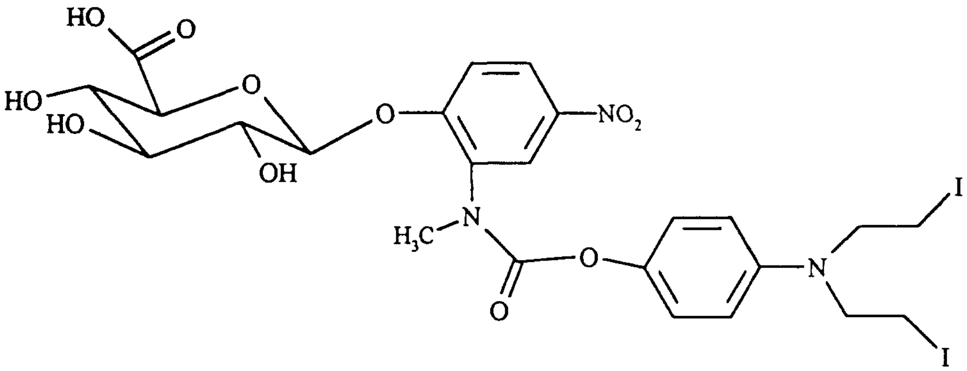

Die Synthese erfolgte analog zu Beispiel 1.

Beispiel 3

(Verbindung 13): Quinin-Prodrug (F 391)

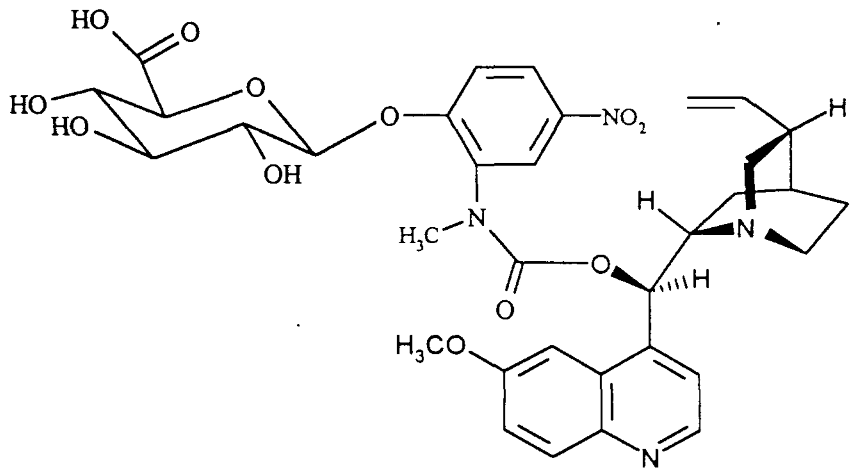

Die Synthese erfolgte analog zu Beispiel 1.

Beispiel 4 (Verbindung 14): Dipyridamol-Prodrug (F 392)

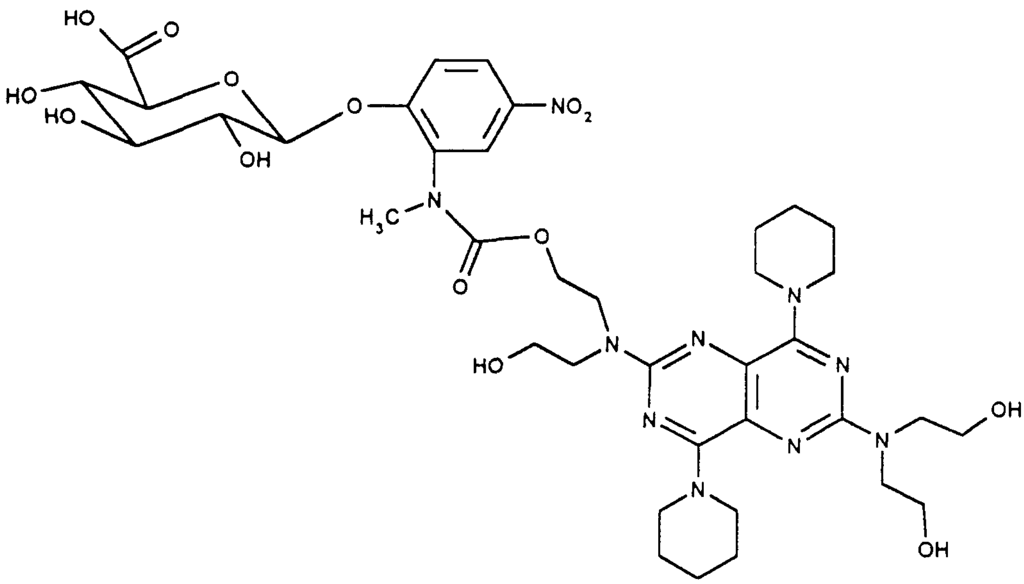

Die Synthese erfolgte analog zu Beispiel 1.

Beispiel 5: Enzymatische Spaltung von F 373

Die Prodrug F 373 (Verbindung 11) wird bei Inkubation mit β-Glucuronidase enzymatisch zu einem aromatischen Stickstofflostderivat (4-[N,N-bis-(2-chloroethyl)amino]phenol) (Verbindung 7), Glucuronsäure und dem Spacer gespalten.

F 373 → Glucuronsäure +

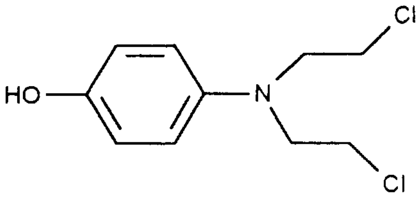

+

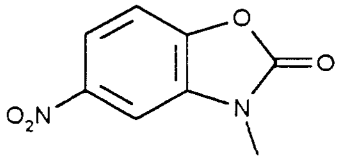

Verbindung 7 Spacer (Verbindung 15)

Die Prodrug F 373 ist in wasserfreiem DMSO in Lösung stabil. Zur Untersuchung der Spaltbarbeit wurden 10 µl F 373-Lösung (5 mg/ml in DMSO) mit 180 µl 0.02 M Phosphatpuffer pH 7.2 und 10 µl E. coli β-Glucuronidase (Sigma) (330 µg/ml) versetzt und bei 37°C inkubiert. Ein 25 µl Ansatz wurde mit 225 µl 0.1 M Phosphatpuffer pH 3.0 (85%) und Acetonitril (15%) verdünnt und sofort mit Hilfe des folgenden HPLC-Systems analysiert.

HPLC System:

Das verwendete HPLC-System bestand aus einer Gradientenpumpe (Gynkotek, Modell 480), einem Autosampler (Abimed, Modell 231/401), einem UV-Detektor (Beckman, Modell 166, Detektionswellenlänge 212 nm) und einer Auswerteeinheit (Beckman, System Gold). Die Trennungen erfolgten auf einer RP- Säule (Zorbax SB-C 18, 5 µm, 125 * 4.6 mm). Die mobile Phase wurde aus zwei Komponenten nach folgendem Schema gebildet:

A - Acetonitril
B - 0.02 M Phosphatpuffer pH 3.0

| | |
|---|---|
| 0 min: | 15 % A, 85 % B |
| 15min: | 75 % A, 25 % B |
| 25 min: | 75 % A, 25 % B |
| 27 min: | 15 % A, 85 % B |
| 35 min: | 15 % A, 85 % B |

Es wurden folgende Peakflächen gefunden:

| Zeit | F 373 | Verbdg. 7 | Spacer Verbd. 15 |
|---|---|---|---|
| min | RT=12,6 | RT=10,7 | RT=14,1 |
| 0 | 19,41 | 0 | 0 |
| 1 | 10,46 | 3,32 | 1,92 |
| 5 | 0,35 | 7,43 | 4,75 |
| 7 | 0 | 7,33 | 5,37 |
| 10 | 0 | 6,79 | 5,43 |
| 15 | 0 | 5,58 | 5,43 |
| 25 | 0 | 4,00 | 5,70 |
| 60 | 0 | 0,88 | 6,53 |

Beispiel 6: Zytotoxizität.von F 373, 391, 392 auf Tumorzellen in An- und Abwesenheit von β-Glucuronidase

In einer 96-Well Mikrotiterplatte wurden 2 x $10^3$ LoVo-Zellen pro well in 100 µl MEM + 10% FKS ausgesät. Nach 24 h wurden die Testsubstanzen in 100 µl Medium in der gewünschten Konzentration und gegebenenfalls zusätzlich β-Glucuronidase (50 µg/ml Endkonzentration; Sigma G 7896) dazugegeben. Jede Gruppe bestand aus 4 wells, die Kontrolle wurde nur mit Medium inkubiert. Nach 65 h wurden 50 µl MTT (2.5 mg/ml in PBS) hinzugegeben und nach 3 h der Überstand entfernt. Der von den lebenden Zellen gebildete Farbstoff wurde durch Zugabe von 100 µl DMSO/well

gelöst. Die Extinktion wurde für jedes well mit Hilfe eines Multiscan Photometers 340 CC (Fa. Flow) bei 492 nm gemessen. Die Werte der 4 wells pro Gruppe wurden gemittelt und daraus die Dosis-Wirkungskurve sowie die $IC_{50}$ mit der Software GraFit 3.0 berechnet.

| Substanz (Prodrug) | ohne β-Gluc. $IC_{50}$ in μmol | mit β-Gluc. $IC_{50}$ in μmol |
|---|---|---|
| F 373 | > 500 | 6.3 |
| F 391 | > 400 | 113 |
| F 392 | > 400 | 49.5 |

Die toxische Verbindung im Prodrug F 373 ist Verbindung 7 (Beispiel 1, Seite 13) und hat alleine getestet eine $IC_{50}$ von 5 μmol. Die toxische Verbindung im Prodrug F 391 ist Quinin und hat alleine getestet eine $IC_{50}$ von 103 μmol. Die toxische Verbindung im Prodrug F 393 ist Dipyridamol und hat alleine getestet eine $IC_{50}$ von 43 μmol.

**Patentansprüche**

1. Verbindung der Formel I

$$\text{Glykosyl-Y[-C(=Y)-X-]}_p\text{-W(R)}_n\text{-Z-C(=Y)-Wirkstoff} \qquad (I)$$

und/oder physiologisch verträgliche Salze der Verbindung der Formel I,
wobei

Glykosyl für ein enzymatisch abspaltbares Poly-, Oligo- oder Monosaccharid steht,
W für für

1) 5- bis 14-gliedriger aromatischer Rest,
2) Naphthyl,
3) Indenyl,
4) Anthryl,
5) Phenanthryl,
6) ein 5- bis 14-gliedriger heterozylischer Rest mit 1, 2, 3 oder 4 Heteroatomen aus der Gruppe Sauerstoff, Stickstoff und Schwefel,
7) $(C_1\text{-}C_6)$-Alkyl,
8) $(C_2\text{-}C_6)$-Alkenyl,
9) $(C_3\text{-}C_6)$-Cycloalkyl oder
10) Phenyl steht,

R für

1) Wasserstoffatom,
2) $(C_1\text{-}C_4)$-Alkyl,
3) Phenyl,
4) Methoxy,
5) Carboxy,
6) Methyloxycarbonyl,
7) -CN,
8) -OH,
9) $-NO_2$,
10) Halogen wie Fluor, Chlor oder Brom,
11) Sulfonyl,
12) Sulfonamid oder
13) Sulfon-$(C_1\text{-}C_4)$-alkylamid steht,

p für Null oder 1 steht,

n für Null, 1, 2 oder 3 steht,
X für

1) Sauerstoffatom,
2) -NH-,
3) Methylenoxy,
4) Methylenamino,
5) Methylen-($C_1$-$C_4$)-alkylamino,
6) ($C_1$-$C_4$)-Alkylamino oder
7) ($C_3$-$C_6$)-Cycolalkylamino steht,

Y für Sauerstoffatom oder -NH- steht,
Z für

1) ($C_1$-$C_4$)-Alkylamino,
2) -N($CH_3$)-,
3) -C($CH_3$)$_2$-NH-,
4) -CH($CH_3$)-NH-,
5) -C($CH_3$)$_2$-N($R^2$)-, worin $R^2$ ($C_1$-$C_4$)-Alkyl bedeutet, oder
6) -NH-, wenn W für ($C_1$-$C_6$)-Alkyl, steht, und

Wirkstoff für eine Verbindung mit biologischer Wirkung steht, die über einen Sauerstoffrest, primären oder sekundärer Aminorest oder einen Iminorest gebunden ist.

**2.** Verbindung der Formel I nach Anspruch 1, wobei Glykosyl für eine enzymatisch abspaltbare Glukuronsäure steht,

W für Phenyl steht,
R für Wasserstoffatom, CN, Nitro, Fluor, Chlor, Brom steht,
p für Null ist,
n für eine ganze Zahl Null, 1 oder 2 steht,
Y für Sauerstoffatom steht,
Z für -N($CH_3$)-, -C($CH_3$)$_2$-NH-, -CH($CH_3$)-NH-, -C($CH_3$)$_2$-N($C_1$-$C_4$)-alkyl-, -CH($CH_3$)-N($C_1$-$C_4$)-alkyl- steht.

**3.** Verbindung der Formel I nach Anspruch 1 oder 2, **dadurch gekennzeichnet, daß** die Verbindung der Formel I
2-[N-Methyl-N-[(4-(N,N'-bis-(2-chloroethyl)amino)phenyloxycarbonyl]amino]-4-nitrophenyl-β-D-glucuronsäure,
2-[N-Methyl-N-[(4-(N,N'-bis-(2-iodoethyl)amino)phenyloxycarbonyl]amino]-4-nitrophenyl-β-D-glucuronsäure,

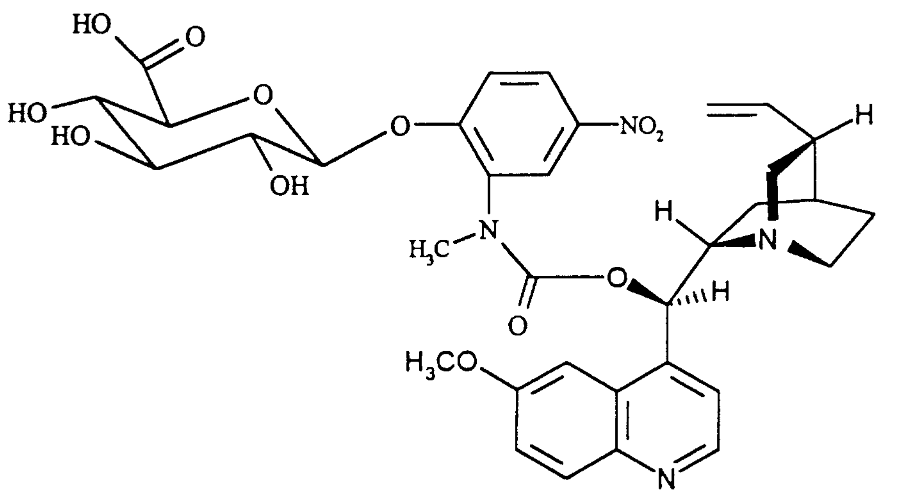

oder

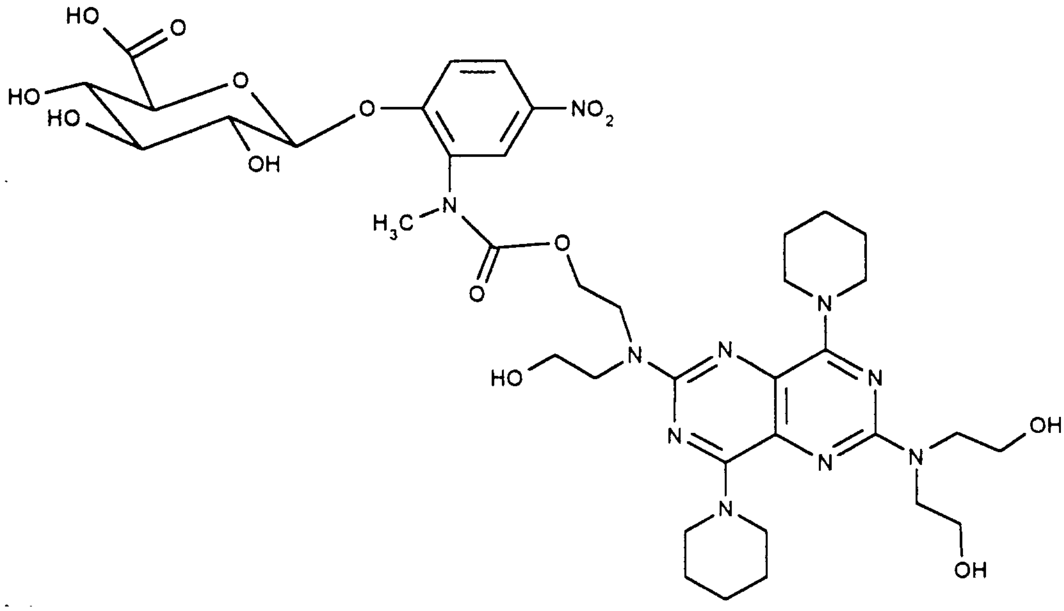

ist.

4. Verfahren zur Herstellung einer Verbindung der Formel I, **dadurch gekennzeichnet, daß** man eine Verbindung der Formel II,

$$\text{Glykosyl-Y[-C(=Y)-X-]}_p\text{-W(R)}_n\text{-Z} \qquad \text{(II)}$$

worin die Reste Glykosyl, Y, X, p, W, R, n und Z die in Formel I nach Anspruch 1 genannte Bedeutung haben, mit einem Wirkstoff, der einen aktivierten Carboxyl-, Amino- oder Iminorest hat, umsetzt,
wobei die Umsetzung in Gegenwart eines Lösungsmittels aus der Gruppe Acetonitril, Dioxan, Tetrahydrofuran, Dichlormethan, Dimethylformamid, Aceton erfolgt und anschließend die Schutzgruppen hydrolytisch abgespalten werden.

5. Arzneimittel, **gekennzeichnet durch** einen wirksamen Gehalt an mindestens einer Verbindung der Formel I nach einem oder mehreren der Ansprüche 1 bis 3, und/oder eines physiologisch verträglichen Salzes der Verbindung der Formel I, wobei die Reste Glykosyl, Y, X, p, W, R, n, Z und Wirkstoff wie in Formel I nach Anspruch 1 definiert sind, zusammen mit einem pharmazeutisch geeignetem und physiologisch verträglichen Trägerstoff, Zusatzstoff und/oder anderen Wirk- und Hilfsstoffen.

6. Verwendung der Verbindung der Formel I nach Anspruch 1 zur Herstellung von Arzneimitteln zur Prophylaxe und Therapie von Krebserkrankungen, Autoimmunerkrankungen und chronisch entzündlicher Erkrankungen wie rheumatische Arthritis.

7. Verfahren zur Herstellung eines Arzneimittels, **dadurch gekennzeichnet, daß** man mindestens eine Verbindung der Formel I nach Anspruch 1 mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

8. Pharmazeutische Zubereitung, enthaltend eine Verbindung der Formel I nach einem oder mehreren der Ansprüche 1 bis 3 und ein Antikörper-Enzym-Konjugat, das den Glykosylrest in der Formel 1 enzymatisch spalten kann.

**Claims**

1. A compound of the formula I

$$\text{glycosyl-Y[-C(=Y)-X-]}_p\text{-W(R)}_n\text{-Z-C(=Y)-active compound} \qquad \text{(I)}$$

and/or physiologically tolerable salts of the compound of the formula I, where

glycosyl is an enzymatically cleavable poly-, oligo- or monosaccharide,
W is

1) a 5- to 14-membered aromatic radical,
2) naphthyl,
3) indenyl,
4) anthryl,
5) phenanthryl,
6) a 5- to 14-membered heterocyclic radical having 1, 2, 3 or 4 heteroatoms from the group consisting of oxygen, nitrogen and sulfur,
7) $(C_1-C_6)$ -alkyl,
8) $(C_2-C_6)$-alkenyl,
9) $(C_3-C_6)$ -cycloalkyl or
10) phenyl,

R is

1) a hydrogen atom,
2) $(C_1-C_4)$-alkyl,
3) phenyl,
4) methoxy,
5) carboxyl,
6) methyloxycarbonyl,
7) -CN,
8) -OH,
9) $-NO_2$,
10) halogen such as fluorine, chlorine or bromine,
11) sulfonyl,
12) sulfonamide or
13) sulfone-$(C_1-C_4)$-alkylamide,

p is zero or 1,
n is zero, 1, 2 or 3,
X is

1) an oxygen atom,
2) -NH-,
3) methylenoxy,
4) methylenamino,
5) methylene-$(C_1-C_4)$-alkylamino,
6) $(C_1-C_4)$-alkylamino or
7) $(C_3-C_6)$-cycloalkylamino,

Y is an oxygen atom or -NH-,
Z is

1) $(C_1-C_4)$-alkylamino,
2) $-N(CH_3)-$,
3) $-C(CH_3)_2-NH-$,
4) $-CH(CH_3)-NH-$,
5) $-C(CH_3)_2-N(R^2)-$, in which $R^2$ is $(C_1-C_4)$-alkyl, or
6) -NH-, if W is $(C_1-C_6)$-alkyl, and

active compound is a compound having biological action, which is bonded via an oxygen radical, primary or secondary aminoradical or an imino radical.

2. A compound of the formula I as claimed in claim 1, where glycosyl is an enzymatically cleavable glucuronic acid,

W is phenyl,
R is a hydrogen atom, CN, nitro, fluorine, chlorine, bromine,
p is zero,
n is an integer zero, 1 or 2,
Y is an oxygen atom,
Z is $-N(CH_3)-$, $-C(CH_3)_2-NH-$, $-CH(CH_3)-NH-$,- $C(CH_3)_2-N(C_1-C_4)alkyl)-$, $-CH(CH_3)-N(C_1-C_4)$ alkyl) -.

3. A compound of the formula I as claimed in claim 1 or 2, wherein the compound of the formula I is 2-[N-methyl-N-[(4-(N,N'-bis(2-chloroethyl)amino)phenyloxycarbonyl]amino]-4-nitrophenyl-β-D-glucuronic acid, 2-[N-methyl-N-[(4-(N,N'-bis(2-iodoethyl)amino)phenyloxycarbonyl]amino]-4-nitrophenyl-β-D-glucuronic acid,

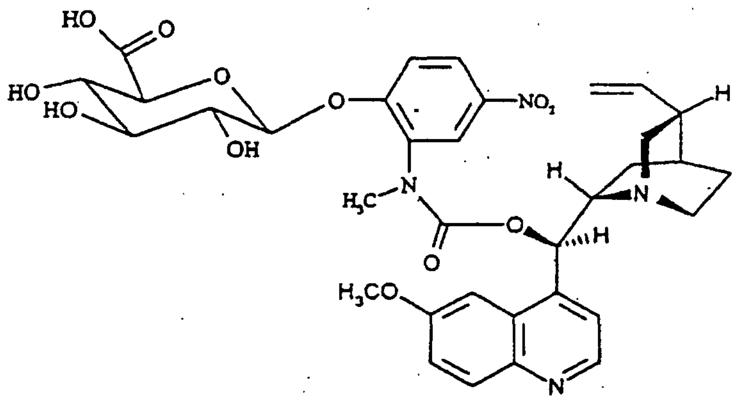

or

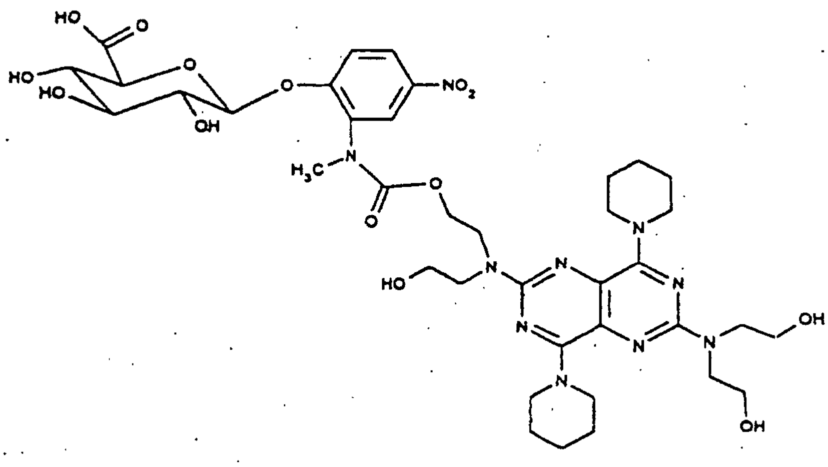

4. A process for the preparation of a compound of the formula I, which comprises reacting a compound of the formula II

$$\text{glycosyl-Y[-C(=Y)-X-]}_p\text{-W(R)}_n\text{-Z} \qquad \text{(II)}$$

in which the radicals glycosyl, Y, X, p, W, R, n and Z have the meaning mentioned in formula I as claimed in claim 1, with an active compound which has an activated carboxyl, amino or imino radical, where the reaction is carried out in the presence of a solvent from the group consisting of acetonitrile, dioxane, tetrahydrofuran, dichloromethane, dimethylformamide, acetone and the protective groups are then removed hydrolytically.

5. A pharmaceutical, comprising an effective amount of at least one compound of the formula I as claimed in one or more of claims 1 to 3, and/or a physiologically tolerable salt of the compound of the formula I, where the radicals

glycosyl, Y, X, p, W, R, n, Z and active compound are as defined in formula I as claimed in claim 1, together with a pharmaceutically suitable and physiologically tolerable excipient, additive and/or other active compounds and auxiliaries.

**6.** The use of the compound of the formula I as claimed in claim 1 for the production of medicaments for the prophylaxis and therapy of carcinomatous diseases, autoimmune diseases and chronic inflammatory diseases such as rheumatoid arthritis.

**7.** A process for the production of a pharmaceutical, which comprises bringing at least one compound of the formula I as claimed in claim 1 into a suitable administration form with a pharmaceutically suitable and physiologically tolerable excipient and, if appropriate, further suitable active compounds, additives or auxiliaries.

**8.** A pharmaceutical preparation comprising a compound of the formula I as claimed in one or more of claims 1 to 3 and an antibody-enzyme conjugate which can enzymatically cleave the glycosyl radical in the formula I.

**Revendications**

**1.** Composés de formule I

$$\text{glycosyl-Y[-C=Y)-X-]}_p\text{-W(R)}_n\text{-Z-C(=Y)- substance active} \qquad (I)$$

et/ou sels physiologiquement acceptables du composé de formule I,
formule dans laquelle

glycosyle représente un poly-, oligo- ou mono- saccharide séparable par voie enzymatique,
W représente

1) un radical aromatique à 5-14 chaînons,
2) le groupe naphtyle,
3) le groupe indényle,
4) le groupe anthryle,
5) le groupe phénanthryle,
6) un radical hétérocyclique à 5-14 chaînons, comportant 1, 2, 3 ou 4 hétéroatomes choisis parmi les atomes d'oxygène, d'azote et de soufre,
7) un groupe alkyle en $C_1$-$C_6$,
8) un groupe alcényle en $C_2$-$C_6$,
9) un groupe cycloalkyle en $C_3$-$C_6$ ou
10) le groupe phényle,

R représente

1) un atome d'hydrogène,
2) un groupe alkyle en $C_1$-$C_4$,
3) le groupe phényle,
4) le groupe méthoxy,
5) le groupe carboxy,
6) le groupe méthyloxycarbonyle,
7) -CN,
8) -OH,
9) $-NO_2$,
10) un atome d'halogène tel que le fluor, le chlore ou le brome,
11) le groupe sulfonyle,
12) le groupe sulfonamido ou
13) un groupe sulfonalkyl($C_1$-$C_4$)amido,

p représente zéro ou 1,
n représente zéro, 1, 2 ou 3,

X représente

1) un atome d'oxygène,
2) -NH-,
3) le groupe méthylène-oxy,
4) le groupe méthylène-amino,
5) un groupe méthylène-alkyl($C_1$-$C_4$)amino,
6) un groupe alkyl($C_1$-$C_4$)amino ou
7) un groupe cycloalkyl($C_3$-$C_6$)amino,

Y représente un atome d'oxygène ou -NH-,
Z représente

1) un groupe alkyl($C_1$-$C_4$)amino,
2) -$N(CH_3)$-,
3) -$C(CH_3)_2$-NH-,
4) -$CH(CH_3)$-NH-,
5) -$C(CH_3)_2$-$N(R^2)$-, où $R^2$ représente un groupe alkyle en $C_1$-$C_4$, et
6) NH, lorsque W représente un groupe alkyle en $C_1$-$C_6$

substance active représente un composé à activité biologique, qui est lié par un atome d'oxygène, un radical amino primaire ou secondaire ou un radical imino.

**2.** Composé de formule I selon la revendication 1, dans lequel

glycosyle représente un acide glucuronique séparable par voie enzymatique,
W représente le groupe phényle,
R représente un atome d'hydrogène, de fluor, de chlore ou de brome ou un groupe CN ou nitro,
p est zéro,
n représente un nombre entier zéro, 1 ou 2,
Y représente un atome d'oxygène,
Z représente -$N(CH_3)$-, -$C(CH_3)_2$-NH-, -$CH(CH_3)$-NH-, -$C(CH_3)_2$-N-alkyle($C_1$-$C_4$)-, -$CH(CH_3)$-N[alkyle($C_1$-$C_4$)]-,

**3.** Composé de formule I selon la revendication 1 ou 2, **caractérisé en ce que** le composé de formule I est l'acide 2-[N-méthyl-N-[(4-(N,N'-bis(2-chloréthyl)-amino) phényloxycarbonyl]amino]-4-nitrophényl-β-D-glucuronique,
l'acide 2-[N-méthyl-N-[(4-(N,N'-bis(2-iodoéthyl)-amino) phényloxycarbonyl]amino]-4-nitrophényl-β-D-glucuronique,

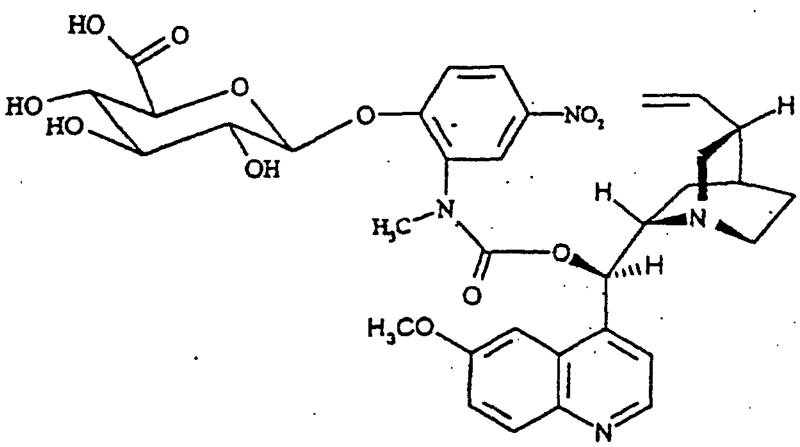

ou

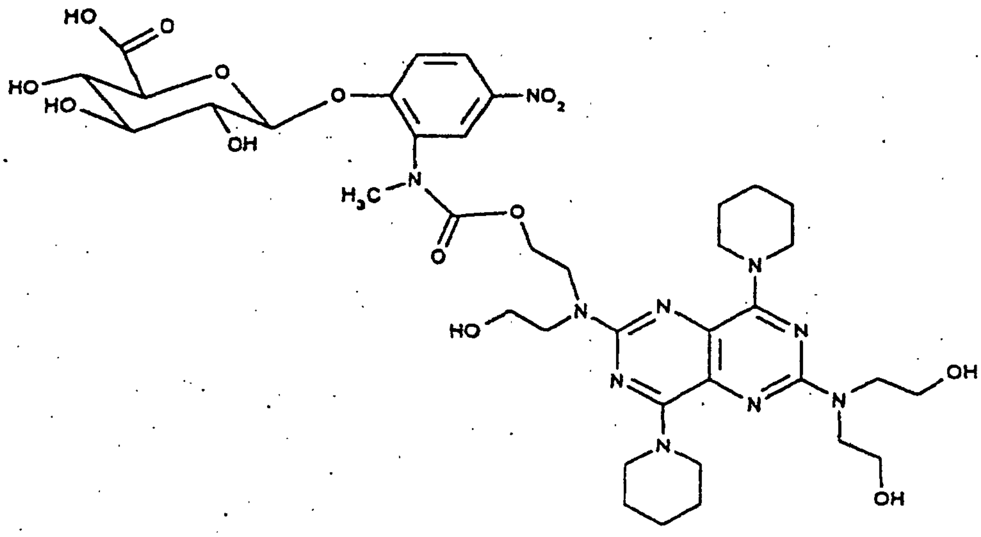

4. Procédé pour la préparation d'un composé de formule I, **caractérisé en ce qu'**on fait réagir un composé de formule II,

$$\text{glycosyl-Y[-C=Y)-X-]}_p\text{-W(R)}_n\text{-Z} \qquad (II)$$

dans laquelle les radicaux Y, X, p, W, R, n et Z ont les significations données dans la formule I selon la revendication 1, avec une substance active qui comporte un radical carboxy, amino ou imino activé,
la réaction s'effectuant en présence d'un solvant choisi dans le groupe constitué par l'acétonitrile, le dioxanne, le tétrahydrofuranne, le dichlorométhane, le diméthylformamide, l'acétone, et on élimine ensuite par hydrolyse les groupes protecteurs.

5. Médicament, **caractérisé par** une teneur efficace en au moins un composé de formule I selon une ou plusieurs des revendications 1 à 3, et/ou en un sel physiologiquement acceptable du composé de formule I, les radicaux glycosyle, Y, X, p, W, R, n, Z et substance active étant tels que définis dans la formule I selon la revendication 1, conjointement avec un véhicule, un additif, pharmaceutiquement appropriés et physiologiquement acceptables et/ou d'autres adjuvants et principes actifs.

6. Utilisation du composé de formule I selon la revendication. 1, pour la fabrication de médicaments destinés à la prophylaxie et au traitement de maladies cancéreuses, de maladies auto-immunes et de maladies inflammatoires chroniques telles que la polyarthrite rhumatoïde.

7. Procédé pour la fabrication d'un médicament, **caractérisé en ce qu'**on met sous une forme d'administration appropriée au moins un composé de formule I selon la revendication 1, avec un véhicule pharmaceutiquement approprié et physiologiquement acceptable et éventuellement d'autres principes actifs, additifs ou adjuvants appropriés.

8. Préparation pharmaceutique, contenant un composé de formule I selon une ou plusieurs des revendications 1 et 3 et un conjugué anticorps-enzyme, qui peut séparer par voie enzymatique le radical glycosyle dans la formule I.